# EUROPEAN PATENT APPLICATION

(11) **EP 4 546 364 A1**
(43) Date of publication of application: **30.04.2025**
(21) Application number: 24206975.5
(22) Date of filing: 16.10.2024
(51) Int. Cl.: G16H 30/40, G16H 50/20, G16H 50/70

(54) **MEDICAL DETERMINATION SUPPORT APPARATUS AND MEDICAL DETERMINATION SUPPORT PROGRAM**

(30) Priority: 27.10.2023 JP 2023184426
(71) Applicant: FUJI-FILM Corporation, Minato-ku Tokyo 106-8620 (JP)
(72) Inventor: ASAFUSA, Katsunori, Tokyo (JP); CHONO, Tomoaki, Tokyo (JP); YOSHIZAWA, Nobuyuki, Tokyo (JP); HATORI, Shohei, Tokyo (JP)
(74) Representative: Dehns Germany Partnerschaft mbB

(57) **Abstract**

A plurality of analysis results for a plurality of analysis items related to a determination target part of a subject are comprehensively determined to reduce a size of a learning model for determining a state of the determination target part.

Medical information received from each medical apparatus (12) is accumulated and stored in a medical information DB (24). An analysis unit (38) analyzes a plurality of analysis items on the basis of a plurality of types of medical information related to a determination target part of a subject which have been acquired by a medical information processing unit (36) and outputs a plurality of analysis results related to the determination target part of the subject for the plurality of analysis items. A comprehensive determination unit (40) determines a state of the determination target part on the basis of an output of a comprehensive determination learning model (30) in a case where the analysis results for the plurality of analysis items related to the determination target part obtained by the analysis unit (38) are input to the comprehensive determination learning model (30) stored in a memory.

## Description

### BACKGROUND OF THE INVENTION

### 1. Field of the Invention

The present specification discloses improvement of a medical determination support apparatus and a medical determination support program.

### 2. Description of the Related Art

In the related art, a doctor determines (for example, diagnoses) a determination target part of a subject on the basis of medical information on the subject acquired by one or a plurality of medical apparatuses. In addition, in the present specification, the determination related to the determination target part means determining the progress or prognosis (for example, prevention prediction, progress prediction, and a risk of onset (including recurrence)) of a certain lesion for the determination target part. Here, in some cases, the doctor performs analysis along various analysis items on the basis of the medical information and comprehensively determines a plurality of analysis results for a plurality of analysis items to determine the determination target part.

For example, in a case where the determination target part is a heart and the determination content is determination related to an ischemic heart disease, the doctor performs analysis along each analysis item, such as coronary artery stenosis, ischemic myocardium, myocardial viability, or heart function, on the basis of medical information on the heart which is the determination target part and comprehensively determines a plurality of analysis results for the plurality of analysis items to determines the ischemic heart disease.

In the related art, an apparatus has been proposed that supports the diagnosis of a subject using a plurality of pieces of medical information (a plurality of pieces of image data). For example, JP4633298B discloses an image diagnosis support system that processes a plurality of pieces of image data of a part to be examined of a subject, which have been acquired by different medical apparatuses (for example, a mammography apparatus and an ultrasound diagnostic apparatus) to generate data for determining the benignancy or malignancy of the part to be examined, generates probability values indicating the probability of a detected lesion being malignant on the basis of feature data of the detected lesion, and calculates a total probability value with reference to a weight corresponding to a combination of the contents of signals indicating the presence or absence of a lesion or a look-up table (LUT). Further, JP2020-192068A discloses an image diagnosis support technique that supports disease discrimination using a plurality of types of measurement values acquired by a medical image acquisition apparatus.

### SUMMARY OF THE INVENTION

Here, it is considered to cause a learning model to perform the comprehensive determination performed by the doctor on the basis of a plurality of analysis results for a plurality of analysis items in the related art. In this case, it is considered that the plurality of analysis results for the plurality of analysis items are input to one learning model and the learning model outputs (prediction of) a determination result based on the plurality of analysis results.

However, this method can cause a problem in that the size of the learning model increases, it is difficult to perform a learning process, and the amount of data processed in a prediction process also increases.

An object of a medical determination support apparatus disclosed in the present specification is to reduce a size of a learning model for comprehensively determining a plurality of analysis results for a plurality of analysis items related to a determination target part of a subject to determine a state of the determination target part.

A medical determination support apparatus disclosed in the present specification includes: an analysis unit that outputs each of a plurality of analysis results related to a determination target part of a subject for a plurality of different analysis items, on the basis of medical information related to the subject; and a comprehensive determination unit that determines a state of the determination target part on the basis of an output of a comprehensive determination learning model, which has been trained to predict the state of the determination target part on the basis of the analysis results for the plurality of analysis items related to the determination target part and to output the state, in a case where the plurality of analysis results output by the analysis unit are input to the comprehensive determination learning model.

The comprehensive determination unit may determine a prognosis of the determination target part on the basis of a prognosis information database, in which state change information indicating a change in the state of the determination target part over time in a past is accumulated and stored, and the determined state of the determination target part.

The medical information may include a plurality of medical images acquired by a plurality of different types of medical apparatuses.

The analysis unit may be configured to include a plurality of analysis learning models that correspond to the analysis items and that have been trained to output analysis results related to the determination target part for the corresponding analysis items on the basis of the medical information.

The determination target part may be a heart, the analysis unit may output a disease state related to each of a plurality of disease types related to the heart as the plurality of analysis results, and the comprehensive determination unit may comprehensively determine a state of the heart of the subject on the basis of the disease state related to each of the plurality of disease types.

The medical determination support apparatus may further include a display control unit that displays the medical information, on which analysis by the analysis unit is based, and a determination result of the comprehensive determination unit on a display unit.

The display control unit may display a screen for selecting either the plurality of pieces of medical information, on which analysis by the analysis unit is based, or the determination result of the comprehensive determination unit on the display unit.

The display control unit may display the medical information in an aspect in which an importance of the medical information is capable of being discriminated on the basis of importance information indicating the importance of each type of the medical information in a case where the analysis result is output.

In addition, a medical determination support program disclosed in the present specification causes a computer to function as: an analysis unit that outputs each of a plurality of analysis results related to a determination target part of a subject for a plurality of different analysis items, on the basis of medical information related to the subject; and a comprehensive determination unit that determines a state of the determination target part on the basis of an output of a comprehensive determination learning model, which has been trained to predict the state of the determination target part on the basis of the analysis results for the plurality of analysis items related to the determination target part and to output the state, in a case where the plurality of analysis results output by the analysis unit are input to the comprehensive determination learning model.

According to the medical determination support apparatus disclosed in the present specification, it is possible to comprehensively determine a plurality of analysis results for a plurality of analysis items related to a determination target part of a subject and to reduce the size of a learning model for determining the state of the determination target part.

### BRIEF DESCRIPTION OF THE DRAWINGS

Fig. 1 is a schematic diagram illustrating a configuration of a medical determination support system according to a present embodiment.
Fig. 2 is a schematic diagram illustrating a configuration of a medical determination support apparatus according to the present embodiment.
Fig. 3 is a functional block diagram illustrating a medical information processing unit.
Fig. 4 is a functional block diagram illustrating an analysis unit.
Fig. 5 is a conceptual diagram illustrating a calculator group for each combination of a type of medical information and an analysis item.
Fig. 6 is a conceptual diagram illustrating the content of importance information.
Fig. 7 is a conceptual diagram illustrating an example of a calculator used for a process for obtaining an analysis result of each analysis item.
Fig. 8 is a conceptual diagram illustrating an aspect in which a calculator is formed by a combination of a structural model and learning information.
Fig. 9 is a diagram illustrating a first display example of a processing result of the medical determination support apparatus.
Fig. 10 is a diagram illustrating a second display example of the processing result of the medical determination support apparatus.

### DESCRIPTION OF THE PREFERRED EMBODIMENTS

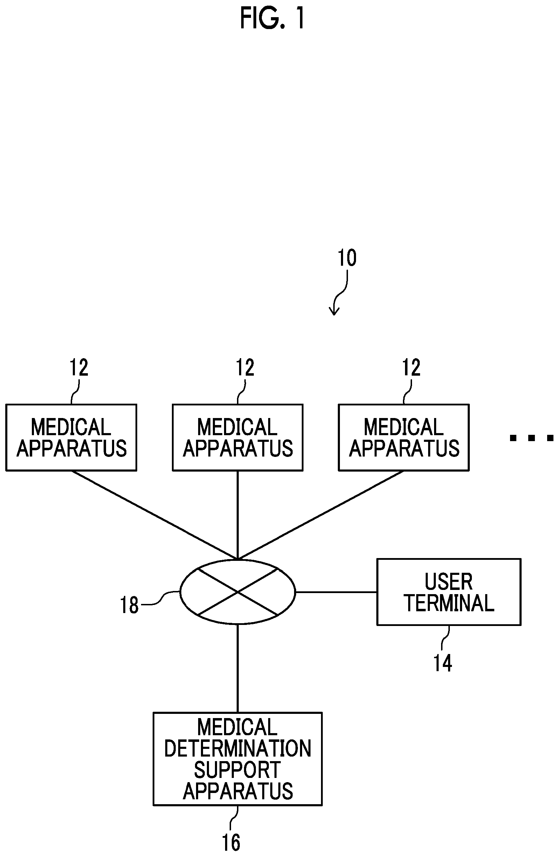

Fig. 1 is a schematic diagram illustrating a configuration of a medical determination support system 10 according to a present embodiment. The medical determination support system 10 is configured to include one or a plurality of medical apparatuses 12, a user terminal 14, and a medical determination support apparatus 16. In the present embodiment, the medical determination support system 10 includes the plurality of medical apparatuses 12. However, the number of medical apparatuses 12 may be one. The plurality of medical apparatuses 12, the user terminal 14, and the medical determination support apparatus 16 are connected via a communication line 18 including a wide area network (WAN) or a local area network (LAN) such that they can communicate with each other.

The medical apparatus 12 is used to acquire medical information of a subject (particularly, a determination target part of the subject). The plurality of medical apparatuses 12 included in the medical determination support system 10 are of different types. For example, the plurality of medical apparatuses 12 include an ultrasound diagnostic apparatus, an X-ray computed tomography (CT) apparatus, a magnetic resonance imaging (MRI) apparatus, a nuclear medicine examination apparatus, a blood tester, a genetic tester, an electrocardiograph, and a cardiac catheter examination apparatus. The medical apparatuses 12 of different types acquire different types of medical information for the determination target part. For example, the ultrasound diagnostic apparatus acquires ultrasound image data of the determination target part, the X-ray CT apparatus acquires CT image data of the determination target part, and the MRI apparatus acquires a nuclear magnetic resonance image of the determination target part. The medical information acquired by each medical apparatus 12 is transmitted to the medical determination support apparatus 16 via the communication line 18.

The user terminal 14 is a terminal used by a user of the medical determination support system 10. The user terminal 14 may be, for example, a personal computer or a tablet terminal. The user terminal 14 is configured to include a processor, a memory, a communication interface, an input interface, and a display.

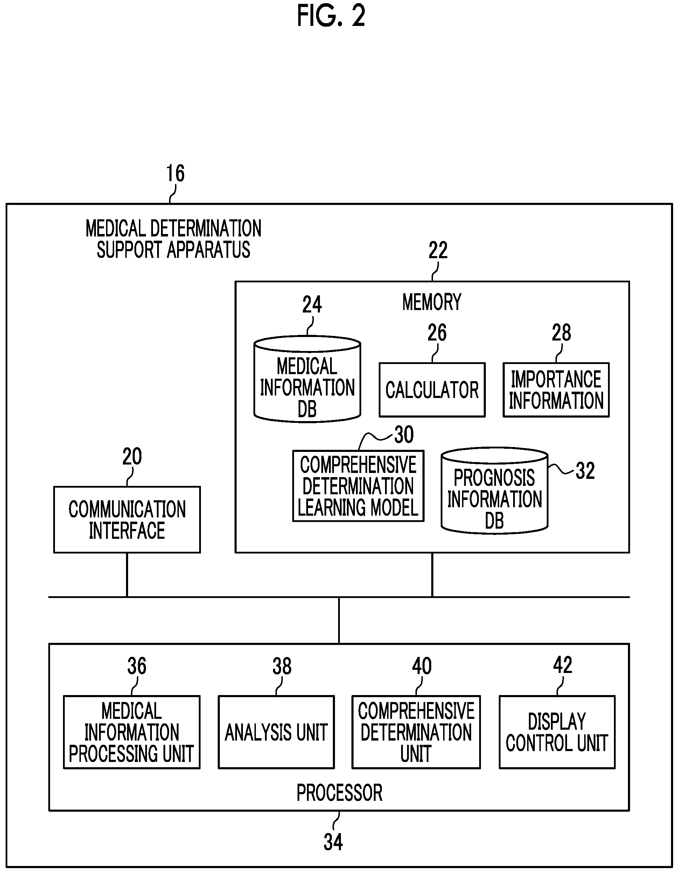

Fig. 2 is a schematic diagram illustrating a configuration of the medical determination support apparatus 16. In the present embodiment, the medical determination support apparatus 16 is a server. However, the medical determination support apparatus 16 may be any apparatus as long as it can exhibit functions that will be described below. In addition, the functions of the medical determination support apparatus 16 that will be described below may be implemented by the cooperation of a plurality of apparatuses that are physically separated from each other.

The communication interface 20 is configured as, for example, a network adapter. The communication interface 20 exhibits the function of communicating with other apparatuses (the medical apparatus 12 and the user terminal 14) via the communication line 18. In particular, in the present embodiment, the communication interface 20 receives the medical information from the plurality of medical apparatuses 12. In addition, the communication interface 20 receives an instruction from the user terminal 14 and transmits information indicating processing results to the user terminal 14.

The memory 22 is configured to include a hard disk drive (HDD), a solid state drive (SSD), an embedded multi media card (eMMC), a read only memory (ROM), a random access memory (RAM), or the like. The memory 22 stores a medical determination support program for operating each unit of the medical determination support apparatus 16. In addition, the medical determination support program can also be stored in a non-transitory computer-readable storage medium such as a Universal Serial Bus (USB) memory or a CD-ROM. The medical determination support apparatus 16 can read the medical determination support program from the storage medium and execute the medical determination support program.

In addition, as illustrated in Fig. 2, a medical information database (DB) 24, a calculator 26, importance information 28, a comprehensive determination learning model 30, and a prognosis information DB 32 are stored in the memory 22. The medical information DB 24 is a database in which the medical information received from each medical apparatus 12 is accumulated and stored. Further, attribute information indicating attributes of the medical information, such as an apparatus ID for uniquely identifying the medical apparatus 12 that has acquired the medical information, a subject ID for uniquely identifying the subject, determination target part information indicating the determination target part, and operation condition information indicating an operation condition (for example, an operation mode) of the medical apparatus 12 in a case where the medical information is acquired, is added to the medical information transmitted from each medical apparatus 12. The medical information and the attribute information are stored in association with each other in the medical information DB 24. In addition, the medical information stored in the medical information DB 24 also includes information (for example, electronic medical records) obtained by, for example, a doctor interviewing the subject.

The calculator 26, the importance information 28, the comprehensive determination learning model 30, and the prognosis information DB 32 will be described below.

The processor 34 is configured to include at least one of a general-purpose processor (for example, a central processing unit (CPU)) or a dedicated processor (for example, a graphics processing unit (GPU), an application specific integrated circuit (ASIC), a field-programmable gate array (FPGA), or a programmable logic device). The processor 34 may be configured by the cooperation of a plurality of processing devices present at positions that are physically separated from each other, instead of one processing device.

As illustrated in Fig. 2, the processor 34 exhibits the functions of a medical information processing unit 36, an analysis unit 38, a comprehensive determination unit 40, and a display control unit 42 according to the medical determination support program stored in the memory 22. The processor 34 exhibits the above-mentioned functions to determine the state of the determination target part of the subject, which will be described in detail below.

First, an outline of the overall process of determining the state of the determination target part of the subject by the processor 34 will be described. The processor 34 receives, from the user terminal 14, determination instruction information including the subject to be determined, the determination target part of the subject, and determination content. In the present embodiment, it is assumed that the determination target part is the heart and the determination content is to determine the state of the heart of the subject, particularly, a state related to an ischemic heart disease (specifically, a lesion state of the ischemic heart disease and prognosis (prevention prediction, progress prediction, a risk of recurrence, and the like)). Of course, the determination target part and the determination content are not limited to the above. In a case where the determination instruction information is received, the processor 34 analyzes a plurality of different analysis items to perform determination for the determination content (in the present embodiment, the determination of the state related to the ischemic heart disease) on the basis of the medical information on the subject stored in the medical information DB 24 and obtains a plurality of analysis results for the plurality of analysis items. In the present embodiment, it is assumed that the plurality of analysis items for determining the state related to the ischemic heart disease are coronary artery stenosis, ischemic myocardium, myocardial viability, heart function, and ischemic heart disease risk. Then, the processor 34 comprehensively determines a plurality of analysis results for the plurality of analysis items to obtain a determination result for the determination content. Hereinafter, details of each function exhibited by the processor 34 will be described.

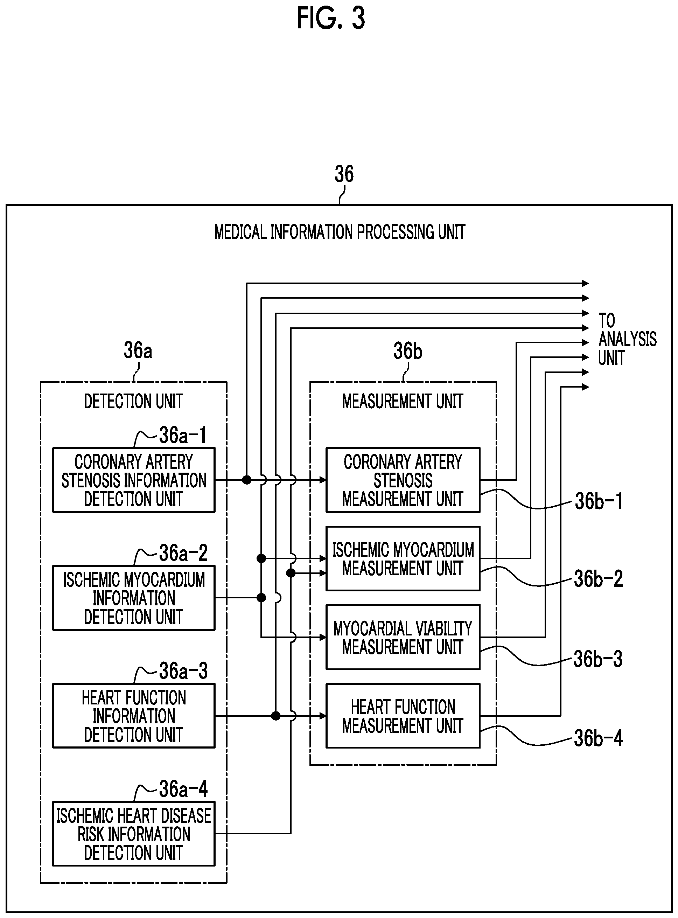

Fig. 3 is a functional block diagram illustrating the medical information processing unit 36. The medical information processing unit 36 performs a process of providing an analysis unit 38, which will be described below, with information necessary for analyzing each analysis item for determining the determination content indicated by the determination instruction information.

First, the medical information processing unit 36 specifies a plurality of different analysis items for determining the determination content indicated by the received determination instruction information. For example, analysis item information, in which the determination content and the plurality of analysis items have been associated with each other is stored in the memory 22 in advance, and the medical information processing unit 36 can specify a plurality of analysis items corresponding to the determination content with reference to the analysis item information.

As illustrated in Fig. 3, the medical information processing unit 36 has the functions of a detection unit 36a and a measurement unit 36b.

The detection unit 36a extracts information necessary for analyzing the plurality of specified analysis items related to the subject indicated by the received determination instruction information from the medical information DB 24. The information necessary for analyzing each analysis item may also be associated in the analysis item information, and the detection unit 36a can specify the information necessary for analyzing each analysis item with reference to the analysis item information and the attribute information associated with the medical information. In Fig. 3, for convenience, each detection unit 36a (a coronary artery stenosis information detection unit 36a-1, an ischemic myocardium information detection unit 36a-2, a heart function information detection unit 36a-3, and an ischemic heart disease risk information detection unit 36a-4) that extracts necessary information is illustrated separately for each analysis item.

The coronary artery stenosis information detection unit 36a-1 extracts information necessary for analyzing the analysis item "coronary artery stenosis" from the medical information DB 24. For example, the coronary artery stenosis information detection unit 36a-1 extracts, from the medical information DB 24, at least one of a resting echocardiographic image, a stress echocardiographic image, or an intravascular ultrasound image (IVUS) acquired by the ultrasound diagnostic apparatus, a coronary CT angiographic (CCTA) image or a fractional flow reserve (FFR)-CT image acquired by the X-ray CT apparatus, a coronary magnetic resonance angiographic (MRA) image or a stress myocardial perfusion MRI image acquired by the MRI apparatus, or a cardiac nuclear medicine examination result acquired by the nuclear medicine examination apparatus.

The ischemic myocardium information detection unit 36a-2 extracts information necessary for analyzing the analysis items "ischemic myocardium" and "myocardial viability" from the medical information DB 24. For example, the ischemic myocardium information detection unit 36a-2 extracts, from the medical information DB 24, at least one of a resting echocardiographic image, a stress echocardiographic image, a myocardial contrast echocardiographic image, or a myocardial strain echocardiographic image acquired by the ultrasound diagnostic apparatus, a dual-energy CT image, a photon counting CT image, a delayed contrast-enhanced CT image, a coronary CT angiographic (CCTA) image, a fractional flow reserve (FFR)-CT image, or a stress perfusion CT image acquired by the X-ray CT apparatus, a cine MRI image, a delayed contrast-enhanced MRI image, or a stress myocardial perfusion MRI image acquired by the MRI apparatus, or a cardiac nuclear medicine examination result acquired by the nuclear medicine examination apparatus.

The heart function information detection unit 36a-3 extracts information necessary for analyzing the analysis item "heart function" from the medical information DB 24. For example, the heart function information detection unit 36a-3 extracts, from the medical information DB 24, at least one of an echocardiographic image acquired by the ultrasound diagnostic apparatus, a cardiac CT image or a fractional flow reserve (FFR)-CT image acquired by the X-ray CT apparatus, a cardiac MRI image or a delayed contrast-enhanced MRI image acquired by the MRI apparatus, or a cardiac nuclear medicine examination result acquired by the nuclear medicine examination apparatus.

The ischemic heart disease risk information detection unit 36a-4 extracts information necessary for analyzing the analysis item "ischemic heart disease risk" from the medical information DB 24. For example, the ischemic heart disease risk information detection unit 36a-4 extracts, from the medical information DB 24, biochemical information and gene factor information acquired from the blood tester, the genetic tester, and the like and information (electronic medical records and the like) obtained by, for example, the doctor interviewing the subj ect.

As described above, the detection unit 36a may acquire a plurality of types of medical information acquired from a plurality of types of medical apparatuses 12 as the information necessary for analyzing each analysis item.

The measurement unit 36b measures parameters necessary for analyzing the plurality of specified analysis items on the basis of the information detected by the detection unit 36a. The parameters necessary for analyzing each analysis item may also be associated in the analysis item information, and the measurement unit 36b can specify the parameters necessary for analyzing each analysis item with reference to the analysis item information. In Fig. 3, for convenience, each measurement unit 36b (a coronary artery stenosis measurement unit 36b-1, an ischemic myocardium measurement unit 36b-2, a myocardial viability measurement unit 36b-3, and a heart function measurement unit 36b-4) that measures necessary parameters is illustrated separately for each analysis item.

The coronary artery stenosis measurement unit 36b-1 measures parameters necessary for analyzing the analysis item "coronary artery stenosis" on the basis of the information extracted by the coronary artery stenosis information detection unit 36a-1. For example, the coronary artery stenosis measurement unit 36b-1 measures at least one of left ventricle ejection fraction (LVEF), left ventricular end-systolic volume, left ventricular global longitudinal strain (GLS, S', mitral annular plane systolic excursion (MAPSE)), coronary artery calcium score (CACS), napkin ring sign, a blood vessel diameter increase value of a lesion part, coronary flow reserve (CFR), or fractional flow reserve (FFR).

The ischemic myocardium measurement unit 36b-2 measures parameters necessary for analyzing the analysis item "ischemic myocardium" on the basis of the information extracted by the ischemic myocardium information detection unit 36a-2 or the ischemic heart disease risk information detection unit 36a-4. For example, the ischemic myocardium measurement unit 36b-2 measures at least one of parameters related to a medical history or physical findings of the subject, parameters related to an exercise electrocardiogram test, parameters related to a Holter electrocardiogram, or parameters related to a heart catheter.

The myocardial viability measurement unit 36b-3 measures parameters necessary for analyzing the analysis item "myocardial viability" on the basis of the information extracted by the ischemic myocardium information detection unit 36a-2. For example, the myocardial viability measurement unit 36b-3 measures at least one of parameters related to myocardial blood flow, wall motion, myocardial fat metabolism, contractile reserve, myocardial fibrosis, myocardial extracellular volume fraction (ECV) measurement, wall thickness, fractional flow reserve (FFR), left ventricle ejection fraction (LVEF), end systolic volume (ESV), end diastolic volume (EDV), myocardial weight, fat degeneration, calcification, a heart catheter, or an electrocardiogram.

The heart function measurement unit 36b-4 measures parameters necessary for analyzing the analysis item "heart function" on the basis of the information extracted by the heart function information detection unit 36a-3. For example, the heart function measurement unit 36b-4 measures at least one of parameters related to left ventricle ejection fraction (LVEF), stroke volume or cardiac output, a left ventricular wall motion score index (WMSI), a left ventricular global longitudinal strain function index (GLS, S', MAPSE), a rate of increase in left ventricular pressure, a left ventricular inflow velocity pattern (a maximum velocity ratio of an early diastolic wave to an atrial systolic wave or the deceleration time of the early diastolic wave), a pulmonary venous flow velocity pattern, a systolic positive wave, an early diastolic positive wave, an atrial systolic negative wave, maximum movement velocity of mitral valve annulus in early diastole, maximum tricuspid regurgitation velocity, systolic pulmonary arterial pressure, right ventricular systolic function, right ventricular diastolic function, myocardial strain analysis, and identification of ischemic/non-ischemic myocardial injury.

In addition, each of the above-described parameters is a known parameter, and a known method can be adopted as a method for measuring each of the parameters. Therefore, details of the method for measuring each of the parameters will be omitted here. In the present specification, it is assumed that the concept of the term "medical information" includes the information necessary for analyzing the plurality of analysis items detected by the detection unit 36a and the parameters for the plurality of analysis items measured by the measurement unit 36b. In the medical information, the information necessary for analyzing the plurality of analysis items detected by the detection unit 36a and the parameters for the plurality of analysis items measured by the measurement unit 36b are particularly described as "medical information acquired by the medical information processing unit 36".

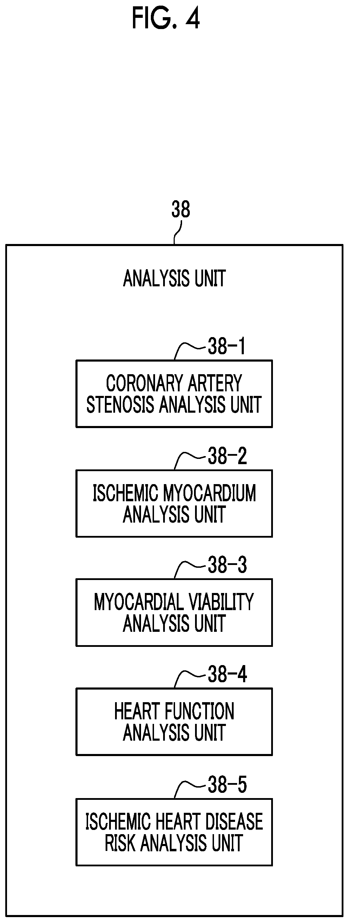

Fig. 4 is a functional block diagram illustrating the analysis unit 38. The analysis unit 38 analyzes a plurality of analysis items on the basis of a plurality of types of medical information related to the subject (particularly, the determination target part of the subject), which have been acquired by the medical information processing unit 36, and outputs a plurality of analysis results related to the determination target part of the subject for the plurality of analysis items. In particular, in the present embodiment, the analysis unit 38 outputs, as the plurality of analysis results, disease states related to each of a plurality of disease types related to the heart which is the determination target part. In Fig. 4, for convenience, each analysis unit 38 (a coronary artery stenosis analysis unit 38-1, an ischemic myocardium analysis unit 38-2, a myocardial viability analysis unit 38-3, a heart function analysis unit 38-4, and an ischemic heart disease risk analysis unit 38-5) that extracts necessary information is illustrated separately for each analysis item.

In the present embodiment, each analysis unit 38 outputs the analysis result using a group of the calculators 26 that corresponds to each of the plurality of types of medical information acquired by the medical information processing unit 36 and performs analysis on the basis of the corresponding type of medical information. In the present specification, the information output by each calculator 26 is referred to as partial analysis information.

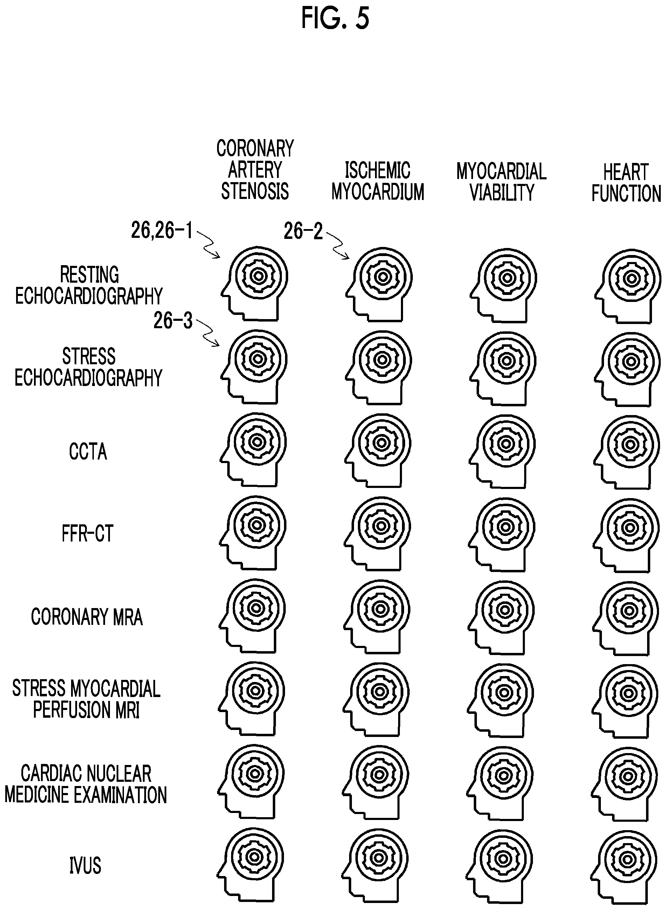

Details will be described with reference to Fig. 5. Fig. 5 is a conceptual diagram illustrating a group of the calculators 26 for each combination of the type of medical information and the analysis item. The group of the calculators 26 illustrated in Fig. 5 is stored in the memory 22. In Fig. 5, a plurality of analysis items (the coronary artery stenosis, the ischemic myocardium, the myocardial viability, and the heart function) are arranged horizontally, and a plurality of types of medical information (resting echocardiography, stress echocardiography, CCTA, FFR-CT, coronary MRA, stress myocardial perfusion MRI, a cardiac nuclear medicine examination, and IVUS) are arranged vertically. In the present embodiment, the plurality of types of medical information illustrated in Fig. 5 include medical information acquired by a plurality of different medical apparatuses 12 (for example, the ultrasound diagnostic apparatus, the X-ray CT apparatus, and the MRI apparatus). In particular, in the present embodiment, the plurality of types of medical information illustrated in Fig. 5 include a plurality of medical images acquired by the plurality of different medical apparatuses 12.

Each calculator 26 illustrated in Fig. 5 corresponds to each analysis item and each type of medical information, and the calculators 26 are different from each other. For example, a calculator 26-1 that outputs partial analysis information related to the coronary artery stenosis on the basis of resting echocardiography, a calculator 26-2 that outputs partial analysis information related to the ischemic myocardium on the basis of resting echocardiography, and a calculator 26-3 that outputs partial analysis information related to the coronary artery stenosis on the basis of stress echocardiography are different calculators 26.

Here, in the present embodiment, each calculator 26 is an analysis learning model. That is, each calculator 26, which is the analysis learning model, is trained in advance to predict partial analysis information for the corresponding analysis item with high accuracy and to output the partial analysis information in a case where the corresponding type of medical information is input. The analysis learning model may be, for example, a neural network. However, the analysis learning model may be any model as long as it exhibits the functions described below. In addition, each calculator 26 does not necessarily need to be a learning model. Each calculator 26 may be any type of calculator as long as it can output the partial analysis information on the corresponding analysis item on the basis of the corresponding type of medical information. For example, each calculator may be a look-up table (LUT).

The coronary artery stenosis analysis unit 38-1 inputs the corresponding types of medical information to a plurality of calculators 26 corresponding to the analysis item "coronary artery stenosis" and each type of medical information (a plurality of calculators 26 arranged vertically corresponding to the analysis item "coronary artery stenosis" in Fig. 5) to obtain a plurality of pieces of partial analysis information. Then, the coronary artery stenosis analysis unit 38-1 calculates an analysis result for the analysis item "coronary artery stenosis" on the basis of the plurality of pieces of partial analysis information output by the plurality of calculators 26.

The ischemic myocardium analysis unit 38-2 inputs the corresponding types of medical information to a plurality of calculators 26 corresponding to the analysis item "ischemic myocardium" and each type of medical information (a plurality of calculators 26 arranged vertically corresponding to the analysis item "ischemic myocardium" in Fig. 5) to obtain a plurality of pieces of partial analysis information. Then, the ischemic myocardium analysis unit 38-2 calculates an analysis result for the analysis item "ischemic myocardium" on the basis of the plurality of pieces of partial analysis information output by the plurality of calculators 26.

The myocardial viability analysis unit 38-3 inputs the corresponding types of medical information to a plurality of calculators 26 corresponding to the analysis item "myocardial viability" and each type of medical information (a plurality of calculators 26 arranged vertically corresponding to the analysis item "myocardial viability" in Fig. 5) to obtain a plurality of pieces of partial analysis information. Then, the myocardial viability analysis unit 38-3 calculates an analysis result for the analysis item "myocardial viability" on the basis of the plurality of pieces of partial analysis information output by the plurality of calculators 26.

The heart function analysis unit 38-4 inputs the corresponding types of medical information to a plurality of calculators 26 corresponding to the analysis item "heart function" and each type of medical information (a plurality of calculators 26 arranged vertically corresponding to the analysis item "heart function" in Fig. 5) to obtain a plurality of pieces of partial analysis information. Then, the heart function analysis unit 38-4 calculates an analysis result for the analysis item "heart function" on the basis of the plurality of pieces of partial analysis information output by the plurality of calculators 26.

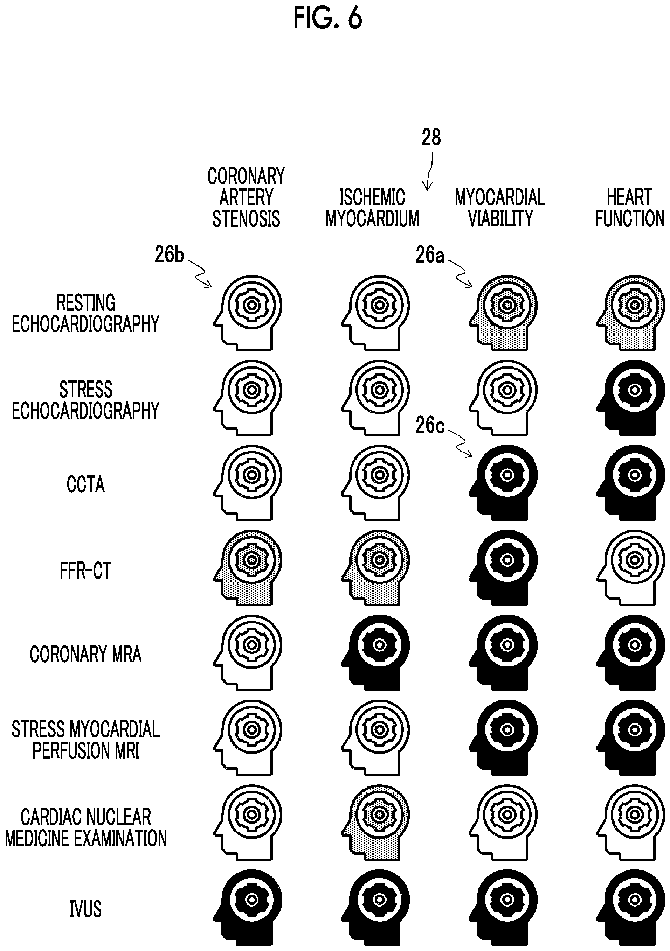

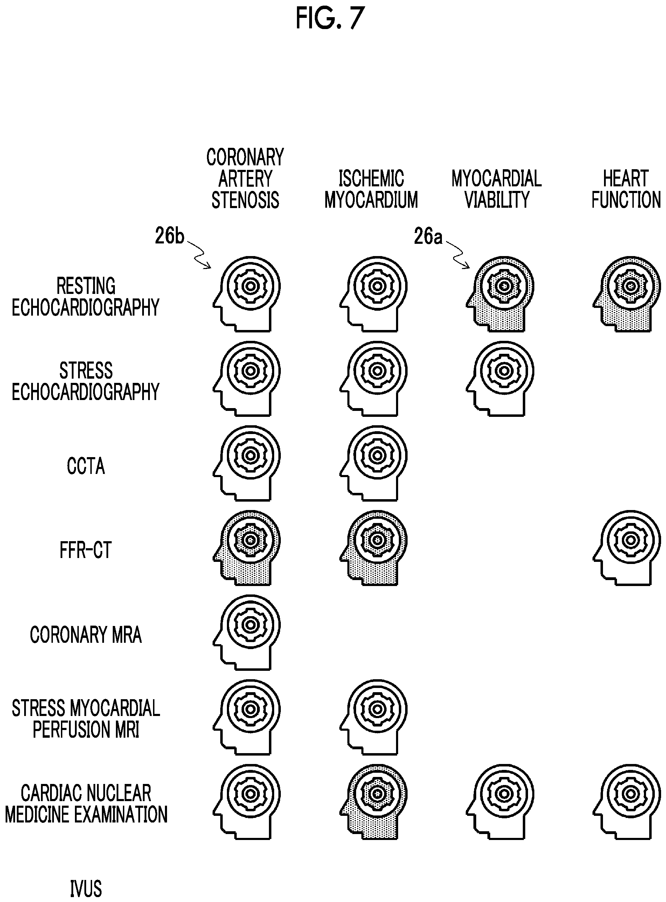

In addition, the calculator 26 used by the ischemic heart disease risk analysis unit 38-5 that outputs the analysis result for the analysis item "ischemic heart disease risk" is not illustrated in Fig. 5 (and Figs. 6 and 7 which will be described below). However, the ischemic heart disease risk analysis unit 38-5 also outputs the analysis result using the calculator (the analysis learning model or the LUT) 26 similarly to the other analysis units 38. Specifically, the ischemic heart disease risk analysis unit 38-5 inputs the information extracted by the ischemic heart disease risk information detection unit 36a-4 to the calculator 26 to analyze the analysis item "ischemic heart disease risk" and outputs the analysis result.

Fig. 6 is a conceptual diagram illustrating the content of the importance information 28 stored in the memory 22. As described above, each analysis unit 38 calculates the analysis result for each analysis item on the basis of a plurality of types of medical information. However, the importance of each type of medical information is different for each analysis item. For example, in a case where the analysis item "coronary artery stenosis" is analyzed, the medical information "FFR-CT" is more important than other types of medical information (that is, the medical information "FFR-CT" has a greater influence on the analysis result than other types of medical information), and the medical information "IVUS" is less important than other types of medical information (that is, the medical information "IVUS" has a smaller influence on the analysis result than other types of medical information).

The importance information 28 is information indicating the importance of each type of medical information in a case where the analysis result for each analysis item is output. In the present embodiment, in the importance information 28, the importance is represented by three levels of "high", "medium", and "low" for each combination of the analysis item and the type of medical information. In Fig. 6, a combination (that is, the calculator 26a) indicated by hatching indicates "high" importance, a combination (that is, the calculator 26b) indicated by white indicates "medium" importance, and a combination (that is, the calculator 26c) indicated by black indicates "low" importance.

For example, in the case of the analysis item "coronary artery stenosis", the importance of the medical information "resting echocardiography", "stress echocardiography", "CCTA", "coronary MRA", "stress myocardial perfusion MRI", and "cardiac nuclear medicine examination" is "medium", the importance of the medical information "FFR-CT" is "high", and the importance of the medical information "IVUS" is "low". On the other hand, in the case of the analysis item "myocardial viability", the importance of the medical information "stress echocardiography" and "cardiac nuclear medicine examination" is "medium", the importance of the medical information "resting echocardiography" is "high", and the importance of the medical information "CCTA", "FFR-CT", "coronary MRA", "stress myocardial perfusion MRI", and "IVUS" is "low". As described above, the importance of a certain calculator 26 (here, referred to as a calculator of interest) and the importance of another calculator 26, which corresponds to the same type of medical information as the calculator of interest, for another analysis item may be different from each other. That is, for the medical information "resting echocardiography", the importance for the analysis item "coronary artery stenosis" is "medium", but the importance for the analysis item "myocardial viability" is "high".

The analysis unit 38 specifies the importance of each piece of medical information (in other words, each calculator 26) with reference to the importance information 28 before the analysis. In the present embodiment, the plurality of analysis units 38 are provided according to the analysis items. Therefore, the analysis unit 38 specifies the importance of each of the plurality of calculators 26 for each analysis item on the basis of the importance information 28. Then, the analysis unit 38 calculates the analysis result for each analysis item on the basis of the partial analysis information output by each calculator 26 in a case where each piece of medical information is input to the corresponding calculator 26 and the specified importance of each calculator 26.

For example, in a case where the content of the importance information 28 is the content illustrated in Fig. 6, since the importance of the medical information "FFR-CT" is "high", the coronary artery stenosis analysis unit 38-1 gives a larger weight (larger than at least the medical information with "medium" importance) to the partial analysis information output by the calculator 26 corresponding to the medical information "FFR-CT" in the calculation of the analysis result for the coronary artery stenosis and then calculates the analysis result for the analysis item "coronary artery stenosis" on the basis of the partial analysis information output by the plurality of calculators 26 corresponding to the plurality of pieces of medical information. In addition, since the importance of the medical information "IVUS" is "low", the coronary artery stenosis analysis unit 38-1 gives a smaller weight (smaller than at least the medical information with "medium" importance) to the partial analysis information output by the calculator 26 corresponding to the medical information "IVUS" in the calculation of the analysis result for the coronary artery stenosis and then calculates the analysis result for the analysis item "coronary artery stenosis" on the basis of the partial analysis information output by the plurality of calculators 26 corresponding to the plurality of pieces of medical information. The other analysis units 38 calculate analysis results in the same manner as described above.

Each analysis unit 38 may calculate the analysis result on the basis of the importance of each piece of specified medical information (that is, each calculator 26) without using some calculators 26 in the group of the calculators 26. For example, as illustrated in Fig. 7, each analysis unit 38 may calculate the analysis result without using the medical information with "low" importance. For example, the myocardial viability analysis unit 38-3 may calculate the analysis result for the analysis item "myocardial viability" using only the medical information "resting echocardiography", "stress echocardiography", and "cardiac nuclear medicine examination". In this case, the myocardial viability analysis unit 38-3 does not use the calculators 26 corresponding to the medical information "CCTA", "FFR-CT", "coronary MRA", "stress myocardial perfusion MRI", and "IVUS" for the calculation of the analysis result.

Here, in some cases, the importance of each type of medical information for each analysis item varies depending on the diagnosis situation (in other words, the state) of the subject (particularly, the determination target part). Therefore, in the importance information 28, the importance of each type of medical information may be associated with the diagnosis situation of the subject. In this case, each analysis unit 38 specifies the importance of each piece of medical information (in other words, each calculator 26) on the basis of the importance information 28 and the diagnosis situation of the subject (particularly, the determination target part) before the analysis.

In the present embodiment, the analysis unit 38 calculates the analysis result using a plurality of calculators 26 corresponding to a plurality of analysis items and a plurality of pieces of medical information. Therefore, as illustrated in Fig. 5 and the like, it is necessary to prepare a large number of calculators 26. In a case where the number of analysis items and the number of types of medical information increase, the number of calculators 26 may become enormous. In particular, in a case where the calculator 26 is an analysis learning model and a large number of calculators 26 are stored in the memory 22 in advance, the storage capacity of the memory 22 is overwhelmed.

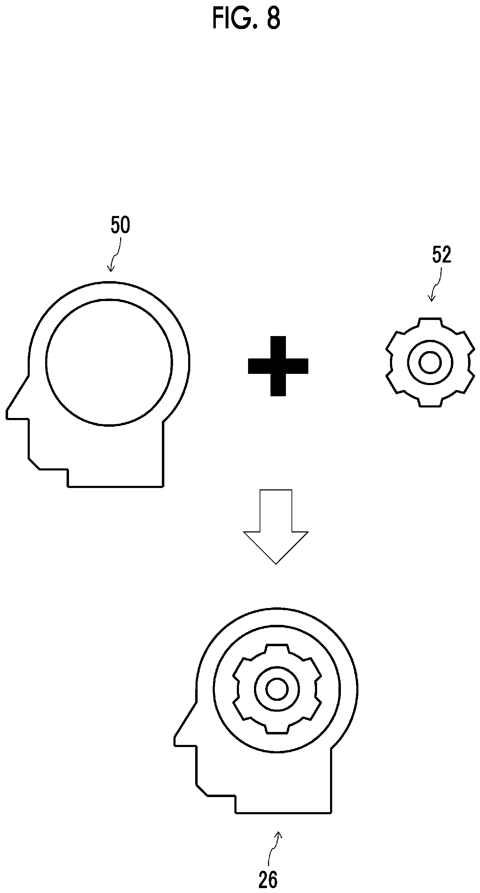

In order to address this problem, in the present embodiment, the calculator 26, which is the analysis learning model, is stored in the memory 22 while being divided into a structural model 50 and learning information 52 as illustrated in Fig. 8. The structural model 50 is data that defines the structure of the analysis learning model (for example, the number of layers of the neural network or the number of neurons in one layer). In other words, the structural model 50 is data defined by hyperparameters (parameters that are not adjusted by a learning process) of the analysis learning model. A plurality of types of structural models 50 may be stored in the memory 22. The learning information 52 is a parameter (for example, a weight for each node of the neural network or a bias of each neuron) that is adjusted by the learning process. The learning information 52 is information obtained by the learning process.

The analysis unit 38 combines the structural model 50 and the learning information 52 to form the calculator 26 which is the analysis learning model. For example, in a case where partial analysis information based on the medical information "resting echocardiography" for the analysis item "coronary artery stenosis" is desired to be obtained, the coronary artery stenosis analysis unit 38-1 combines the structural model 50 for a combination of the analysis item "coronary artery stenosis" and the medical information "resting echocardiography" with the learning information 52 for the combination to form the calculator 26. In addition, in a case where partial analysis information based on the medical information "resting echocardiography" for the analysis item "ischemic myocardium" is desired to be obtained, the ischemic myocardium analysis unit 38-2 combines the structural model 50 for a combination of the analysis item "ischemic myocardium" and the medical information "resting echocardiography" with the learning information 52 for the combination to form the calculator 26.

Since the learning information 52 is different for each combination of the analysis item and the type of medical information, the learning information 52 needs to be individually prepared. The structural model 50 can be used in common for a plurality of combinations of the analysis items and the types of medical information. Therefore, in the configuration in which the structural model 50 and the learning information 52 are stored individually in the memory 22 and the structural model 50 and the learning information 52 are combined to form a necessary calculator 26 in a case where the analysis unit 38 performs the calculation, it is possible to reduce the storage capacity of the memory 22 as compared to at least a case where all of the calculators 26 are stored in the memory 22.

In addition, for the invention in which the analysis unit 38 specifies the importance of each piece of medical information on the basis of the importance information 28 and calculates the analysis result on the basis of the partial analysis information output from each calculator 26 while considering the specified importance, the analysis unit 38 may not necessarily analyze a plurality of analysis items. That is, the invention can be applied even in a case where the number of analysis items analyzed by the analysis unit 38 is one.

Returning to Fig. 2, the comprehensive determination unit 40 determines the state of the determination target part on the basis of the analysis results for a plurality of analysis items related to the determination target part obtained by the analysis unit 38. Specifically, the comprehensive determination unit 40 determines the state of the determination target part on the basis of the output of the comprehensive determination learning model 30 in a case where a plurality of analysis results output by the analysis unit 38 are input to the comprehensive determination learning model 30 stored in the memory 22.

The comprehensive determination learning model 30 may be, for example, a neural network. In addition, the comprehensive determination learning model 30 may be any model as long as it exhibits the functions described below. The comprehensive determination learning model 30 has been trained in advance to predict the state of the determination target part on the basis of the analysis results for a plurality of analysis items related to the determination target part and to output the state. The comprehensive determination learning model 30 is trained by the medical determination support apparatus 16 or a learning device which is another device. For example, the learning device inputs the analysis results for a plurality of analysis items related to the determination target part as learning data to the comprehensive determination learning model 30. The comprehensive determination learning model 30 outputs a prediction result (the state of the determination target part) for the learning data. The learning device calculates a difference between the output of the comprehensive determination learning model 30 and the state of the determination target part as training data and adjusts the parameters of the comprehensive determination learning model 30 such that the difference is reduced. The learning process progresses by repeating this process. The trained comprehensive determination learning model 30 can predict the state of the determination target part with high accuracy on the basis of the analysis results for the plurality of analysis items related to the determination target part and output the predicted state.

As described above, in the present embodiment, the determination target part is the heart, and the analysis unit 38 outputs a disease state for each of a plurality of disease types related to the heart as a plurality of analysis results. Then, the comprehensive determination learning model 30 comprehensively determines the state of the heart, which is the determination target part, on the basis of the disease state for each of the plurality of disease types.

In a case where the medical determination support apparatus 16 does not have the analysis unit 38, all of the medical information acquired by the medical information processing unit 36 is input to the comprehensive determination learning model 30. Even in this case, functionally, the comprehensive determination learning model 30 can predict the state of the determination target part on the basis of the medical information acquired by the medical information processing unit 36 and output the state. However, in this case, the number of inputs to the comprehensive determination learning model 30 becomes enormous, and the amount of calculation required to predict the state of the determination target part also becomes enormous. Therefore, the size of the comprehensive determination learning model 30 also becomes enormous. However, in the present embodiment, the process by the analysis unit 38 is performed before the process of the comprehensive determination learning model 30. Therefore, it is possible to reduce the size of the comprehensive determination learning model 30.

The comprehensive determination unit 40 may determine the prognosis of the determination target part on the basis of the prognosis information DB 32 stored in the memory 22 and the determined state of the determination target part. The prognosis information DB 32 is a database in which state change information indicating changes in the states of the determination target parts (the hearts in the present embodiment) of a plurality of subjects, treatment histories in the past, or the like have been accumulated and stored. The comprehensive determination unit 40 specifies state change information indicating the determined state of the determination target part from the state change information stored in the prognosis information DB 32 and determines the prognosis of the determination target part on the basis of the state of the determination target part after the determined state indicated by the specified state change information. The prognosis of the determination target part includes, for example, a prognosis in a case where the current state is maintained without treatment and a prognosis in a case where appropriate treatment (treatment included in the state change information) is performed.

The display control unit 42 performs a process of displaying the processing result of the medical determination support apparatus 16 on the display unit. For example, the display control unit 42 displays the processing result of the medical determination support apparatus 16 on the display of the user terminal 14. In particular, the display control unit 42 displays the medical information on which the analysis by the analysis unit 38 is based and the determination result of the comprehensive determination unit 40 on the display of the user terminal 14.

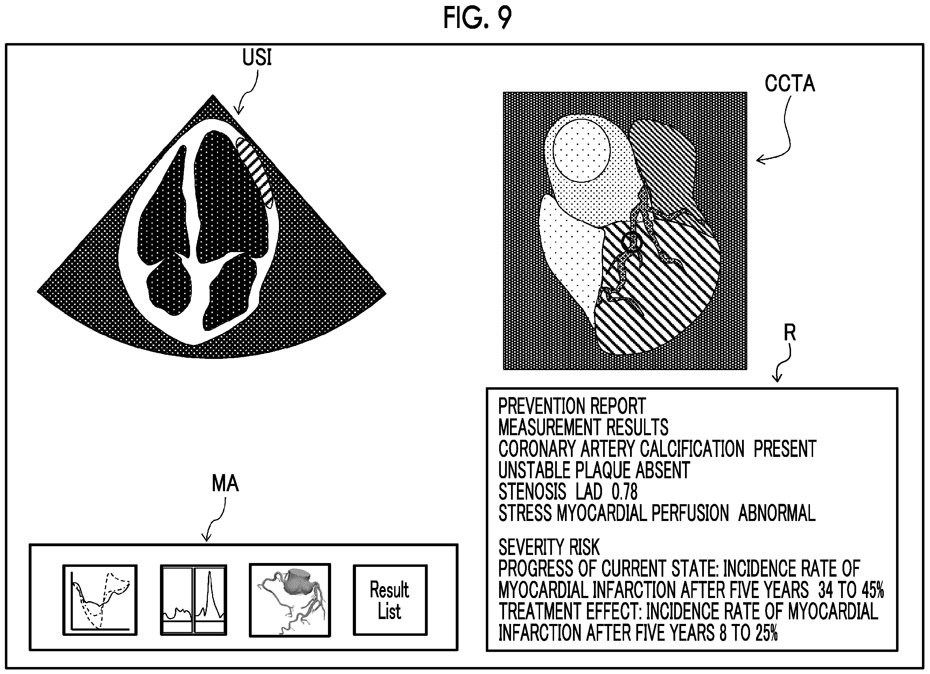

Fig. 9 is a diagram illustrating a first display example of the processing result of the medical determination support apparatus 16. In the example illustrated in Fig. 9, an ultrasound image USI and a coronary CT angiographic image CCTA as the medical information on which the analysis by the analysis unit 38 is based, a report R as the determination result of the comprehensive determination unit 40, and a menu area MA are displayed. The menu area MA is a GUI for the user to input an instruction to select the medical information on which the analysis by the analysis unit 38 is based or the determination result of the comprehensive determination unit 40 or an instruction to display the selected item to be enlarged, and thumbnails or titles are displayed in the menu area MA. For example, the report R shows that there is coronary artery calcification, that there is no unstable plaque, that the stenosis of the left anterior descending artery (LAD) is 0.78, and that there is an abnormality in stress myocardial perfusion as the state of the heart of the subject. Further, the report R shows that the incidence rate of myocardial infarction after five years in the current situation is 34% to 45% and that the incidence rate of myocardial infarction after appropriate treatment is performed is 8% to 25% as a severity risk which is the prognosis predicted by the comprehensive determination unit 40.

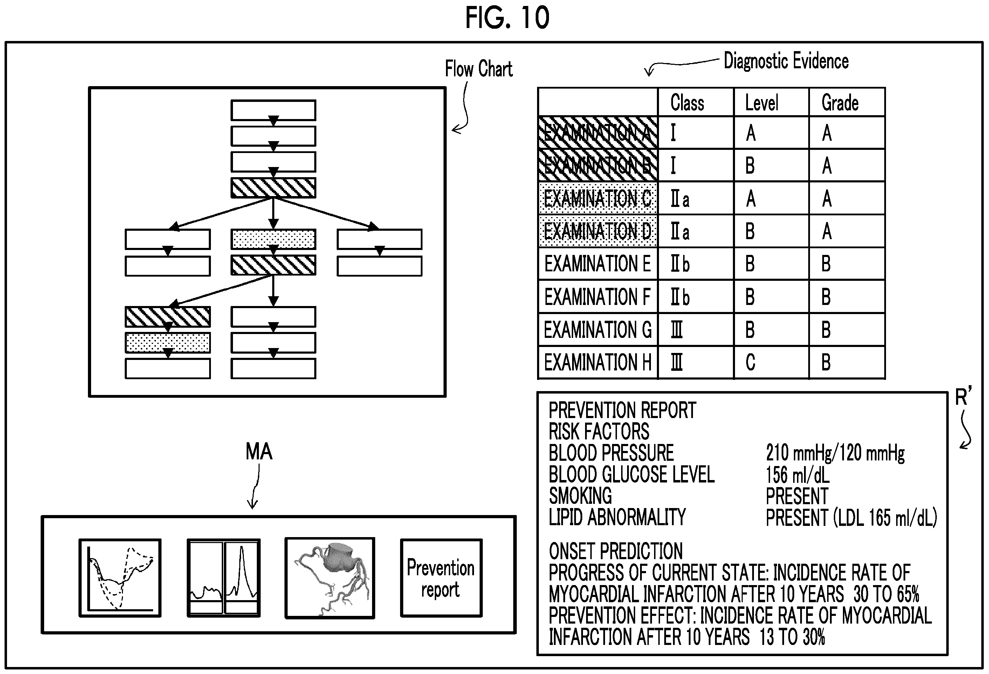

Fig. 10 is a diagram illustrating a second display example of the processing result of the medical determination support apparatus 16. In the example illustrated in Fig. 10, an examination flow for obtaining the medical information on which the analysis by the analysis unit 38 is based and diagnostic evidence (a class, a level, and a grade) are displayed, and a report R' is displayed as the determination result of the comprehensive determination unit 40. In particular, the examination flow for obtaining the medical information related to the importance information 28 or the diagnostic evidence may be displayed in an aspect in which the importance of the medical information can be discriminated by highlight display or the like. In addition, the display order of the diagnostic evidence may be sorted from the top to the bottom in descending order of importance. In the report R', blood pressure, a blood glucose level, a smoking habit, the presence or absence of lipid abnormality, and the like are shown as risk factors for the onset. Further, the report R' shows that the incidence rate of myocardial infarction after 10 years in the current situation is 30% to 65% and that the incidence rate of myocardial infarction in a case where appropriate prevention is performed is 13% to 30% as the onset prediction by the comprehensive determination unit 40.

The embodiment according to the present invention has been described above. However, the present invention is not limited to the above-described embodiment, and various modifications can be made without departing from the gist of the present invention.

### Explanation of References

- 10:: medical determination support system
- 12:: medical apparatus
- 14:: user terminal
- 16:: medical determination support apparatus
- 18:: communication line
- 20:: communication interface
- 22:: memory
- 24:: medical information DB
- 26:: calculator
- 28:: importance information
- 30:: comprehensive determination learning model
- 32:: prognosis information DB
- 34:: processor
- 36:: medical information processing unit
- 38:: analysis unit
- 40:: comprehensive determination unit
- 42:: display control unit

## Claims

1. A medical determination support apparatus (16) comprising:
an analysis unit (38) that outputs each of a plurality of analysis results related to a determination target part of a subject for a plurality of different analysis items, on the basis of medical information related to the subject; and
a comprehensive determination unit (40) that determines a state of the determination target part on the basis of an output of a comprehensive determination learning model (30), which has been trained to predict the state of the determination target part on the basis of the analysis results for the plurality of analysis items related to the determination target part and to output the state, in a case where the plurality of analysis results output by the analysis unit (38) are input to the comprehensive determination learning model (30).

2. The medical determination support apparatus (16) according to claim 1,
wherein the comprehensive determination unit (40) determines a prognosis of the determination target part on the basis of a prognosis information database, in which state change information indicating a change in the state of the determination target part over time in a past is accumulated and stored, and the determined state of the determination target part.

3. The medical determination support apparatus (16) according to claim 1,
wherein the medical information includes a plurality of medical images acquired by a plurality of different types of medical apparatuses (12).

4. The medical determination support apparatus (16) according to claim 1,
wherein the analysis unit (38) is configured to include a plurality of analysis learning models that correspond to the analysis items and that have been trained to output analysis results related to the determination target part for the corresponding analysis items on the basis of the medical information.

5. The medical determination support apparatus (16) according to claim 1,
wherein the determination target part is a heart,
the analysis unit (38) outputs a disease state related to each of a plurality of disease types related to the heart as the plurality of analysis results, and
the comprehensive determination unit (40) comprehensively determines a state of the heart of the subject on the basis of the disease state related to each of the plurality of disease types.

6. The medical determination support apparatus (16) according to claim 1, further comprising:
a display control unit (42) that displays the medical information, on which analysis by the analysis unit (38) is based, and a determination result of the comprehensive determination unit (40) on a display unit.

7. The medical determination support apparatus (16) according to claim 6,
wherein the display control unit (42) displays a screen for selecting either the plurality of pieces of medical information, on which analysis by the analysis unit (38) is based, or the determination result of the comprehensive determination unit (40) on the display unit.

8. The medical determination support apparatus (16) according to claim 6,
wherein the display control unit (42) displays the medical information in an aspect in which an importance of the medical information is capable of being discriminated on the basis of importance information indicating the importance of each type of the medical information in a case where the analysis result is output.

9. A non-transitory computer-readable storage medium storing a medical determination support program causing a computer to function as:
an analysis unit (38) that outputs each of a plurality of analysis results related to a determination target part of a subject for a plurality of different analysis items, on the basis of medical information related to the subject; and
a comprehensive determination unit (40) that determines a state of the determination target part on the basis of an output of a comprehensive determination learning model (30), which has been trained to predict the state of the determination target part on the basis of the analysis results for the plurality of analysis items related to the determination target part and to output the state, in a case where the plurality of analysis results output by the analysis unit (38) are input to the comprehensive determination learning model (30).
